# EUROPEAN PATENT APPLICATION

(11) **EP 3 758 005 A1**
(43) Date of publication of application: **30.12.2020**
(21) Application number: 19181994.5
(22) Date of filing: 24.06.2019
(51) Int. Cl.: G16B 25/10, C12Q 1/6883, C12Q 1/6886

(54) **IDENTIFICATION OF THE CELLULAR FUNCTION OF AN ACTIVE NFKB PATHWAY**

(71) Applicant: Koninklijke Philips N.V., 5656 AG Eindhoven (NL)
(72) Inventor: VAN DOORN, Arie Rombertus, 5656 AE Eindhoven (NL); VAN DE STOLPE, Anja, 5656 AE Eindhoven (NL)
(74) Representative: de Haan, Poul Erik

(57) **Abstract**

Target genes of the NFkB cellular signaling pathway are disclosed, which are associated with a cellular function such as cell division, apoptosis or protection against oxidative stress of an active NFkB cellular signaling pathway and can be used for determining a cellular function of an active NFkB cellular signaling in a human subject.

## Description

### FIELD OF THE INVENTION

The present invention generally relates to the field of bioinformatics, genomic/transcriptomic processing, proteomic processing, and related arts. More particularly, the present invention relates to certain target genes of the NFkB cellular signaling pathway, which are associated with a cellular function of an active NFkB cellular signaling pathway and can be used for determining a cellular function of an active NFkB cellular signaling in a subject. The present invention also relates to a method for determining a cellular function of an active NFkB cellular signaling pathway in a cell sample, the method comprising determining in the cell sample containing cells with an active NFkB cellular signaling pathway the cellular function based on expression level of at least one NFkB target gene associated with the cellular function. The present invention further relates to a kit comprising components for determining the expression levels of at least one NFkB target gene associated with a cellular function. The present invention further relates to an apparatus comprising a digital processor configured to perform the method, a non-transitory storage medium storing instructions that are executable by a digital processing device to perform such a method, and a computer program comprising program code means for causing a digital processing device to perform such a method.

### BACKGROUND OF THE INVENTION

An appropriately functioning immune system is crucial for maintaining health and limiting the damage of disease. An appropriate immune response protects against disease, is relevant for the course of a disease and may be required for optimal effect of therapeutics.

The immune system is made up by a large number of immune cell types that work together in a coordinated manner to produce the right immune response to, for example, an invading pathogen or an internal disease like cancer. A distinction is made between the innate immune system which controls early inflammatory responses, and the adaptive immune response that controls long term immune responses.

The mechanistic principle behind its functioning is that the immune system generates an immune response to non-self antigens, like an infectious agent or an abnormal protein on a cancer cell. Recognition of such antigens is at the core of a functioning immune system. After an elaborate process in which non-self antigens are recognized as non-self, effector T cells are instructed to find and recognize the specific antigen and attack the invader carrying the antigen.

The right balance between overactivity and underactivity of the system is crucial for development of disease and maintenance of health and correction of the balance is an important therapeutic approach.

Both overactivity as well as inactivity can lead to disease, and multiple drugs are available and being developed to correct a defect in the immune response causing a specific disease, either to increase the activity of the immune system, like in cancer or infections, or to reduce its activity, like in auto-immune disease, or allogenic transplantations.

Predicting and monitoring which therapy is most effective is difficult, also in the case of (drug-based) therapies which specifically target a cellular mechanism underlying the abnormal functioning of the immune system.

A few example diseases in which the immune response is therapeutically modulated in order to treat the disease are cancer, kidney transplantation, rheumatoid arthritis, psoriasis and diabetes.

Especially for treatment of cancer patients using immunotherapy, a lot of progress has been made recently and many drugs have been, and are being developed for this purpose. In case of cancer, in addition to effects of changes in the genome of cancer cells, cancer growth and metastasis are influenced by the cancer cell microenvironment, mainly consisting of fibroblasts and cells of the immune system. Infiltration of cancer tissue by immune cells, both of the monocyte macrophage lineage and a variety of lymphocyte subtypes plays an important role, either in mounting an immune response (appropriately active antigen-presenting dendritic cells, cytotoxic T cells and CD4+ helper T cells) or creating immune tolerance to the cancer cells (regulatory/suppressor T cells, inhibition of activity of immune cells by production of IL10 or TGF-beta), or even promoting tumor progression. Thus, immune cell infiltration can have both tumor suppressive as well as tumor promoting effects, depending on the immune subtypes present, their functional status and the type of cancer cell.

In general, in cancer, especially in advanced cancer, the immune response has failed, either because it is not aware of cancer cells present, or it has become exhausted ("tolerant") by continuous interaction with cancer antigens.

Immunotherapy against cancer aims at respectively enabling or restoring an effective anti-cancer immune response. In the first case when the immune system was not aware of the cancer being present, full cure of cancer may in principle be possible once an effective immune response is generated, for example by vaccination with cancer antigens, and therapy duration is limited. In contrast, in the case of an exhausted immune system cure will in general not be possible, which necessitates continuation of therapy as long as possible, in this case by interfering with the immune suppressive (inter)actions of cancer cells and various immune cells, especially CD8+ T cells, by checkpoint inhibitor drugs. A large number of immunotherapy drugs which activates the immune response in a targeted manner is in development, for example by blocking PD1 - PDL1 (immune activity checkpoint) interaction and signaling.

Many other diseases, in particular (auto)immuno-mediated diseases and immunodeficiency diseases are also treated with immunomodulatory drugs. In the first case the immune response needs to be dampened; in the second case its effectiveness needs to be increased. In all cases it is important that the effect of the drug is as specific in correcting the defect as possible. Also here a large number of drugs are being developed, with often facing the same challenges as described for cancer.

In immunotherapy (for all diseases) there are a number of clinical challenges: (1) predict therapy response in the individual patient, since only a small percentage will respond; this also includes prediction of response to combination immunotherapy versus monotherapy, in case of cancer also in vivo vaccination by inducing release of antigens from the cancer cells (e.g. by radiation), and other vaccination therapies; (2) assess as soon as possible whether a therapy is effective in view of side effects and costs; (3) identify patients that are at risk of severe side-effects of immunotherapy. Neither of the challenges has been adequately addressed. For example for cancer, quantification of CD8+ and CD3/4+ cells is available as well as PD1 and PD-L1 staining to predict therapy response, however neither one is reliable. Therapy response assessment is generally possible about 3 to 6 months after initiation of therapy.

There is thus a high need for biomarker-based assays which can predict and assess/monitor therapy response to specific immunotherapy drugs or drug/therapy combinations, in case of cancer also including combinations between immunotherapy drugs and other therapies which induce release of antigens from cancer cells, like radiation, chemotherapy and targeted therapy.

Nuclear factor-kappa B (nuclear factor kappa-light-chain-enhancer of activated B cells, NFkB, NFκB or NFKB) is an inducible transcription factor that regulates the expression of many genes involved in the immune response. The NFkB pathway is a key cellular signaling pathway involved in immune, inflammatory, and acute phase response, but is also implicated in the control of cell survival, proliferation, and apoptosis. In healthy, non-activated cells, the NFkB cellular signaling pathway associated transcription factors that are composed of dimers originating from five genes (NFKB 1 or p50/p105, NFKB2 or p52/p100, RELA or p65, REL, and RELB) are predominantly cytoplasmic due to their interaction with the inhibitors of NFkB (IkBs) and therefore remain transcriptionally inactive, thus keeping the NFkB cellular signaling pathway inactive. The NFκB transcription factor can be activated by a large variety of stimuli, like reactive oxygen species, pathogens, cytokines, and by activation of T- and B-cell receptors in immune cells. Upon activation the IkBs become phosphorylated and undergo ubiquitin-dependent degradation by the proteasome, and NFkB dimers are translocated to the nucleus where they act as transcription factors. Activity of the NFkB cellular signaling pathway is known to be associated with different types of cancer and to support pro-survival phenotypes of cancer cells.

WO 2017/029215 A1 discloses a computational model for inferring activity of the NFκB pathway, based on measurement of transcription factor target gene levels. However, it remains unknown which cellular function is associated with it, if the NFκB pathway was inferred to be active. NFκB pathway can exert multiple cellular functions including cell division, cell differentiation, cell adhesion, chemotaxis and migration, apoptosis, immune functions like antigen processing, and generation of and protection against oxidative stress, which functions are determined by the cell type, and the cellular condition as determined by intracellular and extracellular signals.

It would thus be desirable to not only know whether the NFκB pathway is active or inactive but also more specifically know which cellular function is associated with an active NFκB pathway. That is, if an active NFkB pathway was detected, it would be of importance to identify the function of the NFkB pathway in that sample. For example, if the NFkB pathway was measured to be active in a cancer tissue sample, but it would have a functional state to enhance the immune response, blocking the NFkB activity would probably be contraindicated; on the other hand, if NFkB activity was associated with oxidative stress in a cancer sample which supports cancer progression, NFkB would likely provide a clinically relevant drug target in the respective patient.

The present invention is concerned with the problem of providing an enhanced diagnosis for immune-related conditions.

### SUMMARY OF THE INVENTION

The above problem is solved by a target gene or a set of target genes of the NFkB cellular signaling pathway, which is / are associated with a cellular function of an active NFkB cellular signaling pathway (herein also referred to as cellular function-associated NFkB target gene (FATG) or genes (FATGs)).

In accordance with a main aspect of the present invention, a method for determining a cellular function of an active NFkB cellular signaling pathway in a cell sample is provided. The method comprises the step of determining in the cell sample containing cells with an active NFkB cellular signaling pathway the cellular function based on expression level of at least one NFkB target gene associated with the cellular function.

The present invention is based on the realization that specifc NFkB target genes are differentially expressed between cells in which different cellular functions are exerted by an active NFkB cellular signaling. These FATGs can thus be used for determining a cellular function of an active NFkB cellular signaling in a subject.

The term "target gene", as used herein, means a gene whose transcription is directly or indirectly controlled by a respective transcription factor element. The "target gene" may be a "direct target gene" and/or an "indirect target gene". An NFkB target gene is in particular a gene associated with the NFKB cellular signaling pathway and differentially expressed between an NFKB cellular signaling pathway exerting the respective function and an NFKB cellular signaling pathway not exerting the respective function. An NFkB target gene is understood herein to be associated with a cellular function of an active NFkB cellular signaling pathway if it is differentially expressed between cells with the respective cellular function being active and cells with the cellular function being inactive. While already one FATG may be indicative for a particular cellular function, the present invention envisages the use of more than one FATG, e.g. 2, 3, 4, 5, 6, 7, 8, 9, 10, 11, 12, 13 or even 14 FATGs. The expression level may be based on the amount of transcribed RNA or protein content, preferably the amount of RNA, in particular mRNA.

The phrase "expression level of a target gene" denotes a value that is representative of the amount, e.g. a concentration, of a target gene present in a sample. This value may be based on the amount of a transcription product of the target gene (e.g. mRNA) or a translation product thereof (e.g. protein). Preferably, the expression level is based on the amount of mRNA formed from the target gene. In order to determine the expression level, techniques such as qPCR, multiple qPCR, multiplexed qPCR, ddPCR, RNAseq, RNA expression array or mass spectrometry may be used. For example, a gene expression microarray, e.g. Affymetrix microarray, or RNA sequencing methods, like an Illumina sequencer, can be used.

Activity of the NFkB pathway may be determined in accordance with the method described in WO 2017/029215 A1, which is incorporated herein by reference in its entirety, in particular for the purpose of determining whether the NFkB cellular signaling pathway is active and/or inferring (e.g. quantifying) the activity of the NFkB cellular signaling pathway. Particular reference is made to the target genes and corresponding sequence listings described therein. For the purposes of the present invention, an NFkB cellular signaling pathway is considered to be active if IkBs are determined to be phosphorylated, and/or IkBs are determined to be absent (e.g. degraded), and/or NFkB dimers are determined to be present in the nucleus and/or NFkB dimers are determined to be absent in the cytoplasm and/or the activity of the NFkB pathway, as determined by pathway analysis as described herein and/or in WO 2017/029215 A1, is determined to be active based on the NFkB pathway activity score. This score, as described in WO 2017/029215 A1 indicates a level of pathway activity, in principle without a certain defined threshold between active and inactive. The higher the pathway activity score, the more active the NFkB pathway is in a certain measured sample. This measured level of activity can have various functions, which can be defined by the here described method. By combining activity with the function determination it is possible to obtain information how strong cells exert a respective NFkB function.

The at least one NFkB target gene is selectable based on differential expression studies comparing an expression level of the at least one cellular function-associated NFkB target gene of an active NFkB cellular signaling pathway, whose respective cellular function is active, with an expression level of the at least one cellular function-associated NFkB target gene of an active NFkB cellular signaling pathway, whose respective cellular function is inactive and/or whose NFkB signaling pathway is substantially inactive (reference expression). According to a preferred embodiment, the at least one NFkB target gene (FATG) is selected from the group consisting of BCL2L1, BCL2, CCND2, SKP2, NOX1, SERPINE2, CSF2, CYP27B1, IGHG1, IGHE, BIRC3, CCL2, SOD2, GZMB, CCL5 and MMP1. Preferably, the at least one NFkB target gene (FATG) is selected from the group consisting of BCL2L1, BCL2, CCND2, SKP2, CYP27B1, IGHG1, IGHE, BIRC3, CCL2, SOD2 and GZMB.

Following differential expression studies between cells having a known cellular function and corresponding cells, whose respective cellular function is known to be inactive and/or whose NFkB signaling pathway is substantially inactive, as described above, additional FATGs may be identified. In particular, it is conceivable that following collection of immune cell data in the future, using this method, the list of suitable FATGs can be enhanced based on data analysis as described herein, and target gene combinations selected that further improve the function of the here described NFkB pathway-associated cellular function analysis.

The term "cellular function" is interchangeably used herein with the terms functional status and functional state, and describes a phenotype (function) that is at least partially linked to differential expression of the at least one NFkB target gene associated with the cellular function.

According to a preferred embodiment, the cellular function is selected from the group consisting of cell division, cell differentiation, cell adhesion, chemotaxis and migration, apoptosis, immune functions like antigen presentation, oxidative stress and oxidative stress protection. In particular, the cellular function is selected from the group consisting of cell division, apoptosis and oxidative stress protection. Target genes associated with cell division are preferably selected from the group consisting of BCL2, BCL2L1, CCND2, SKP2, CSF1, NOX1 and SERPINE2, and in particular selected from the group consisting of BCL2, BCL2L1, CCND2 and SKP2. Target genes associated with cell apoptosis are preferably selected from the group consisting of CYP27B1, IGHE and IGHG1. Target genes associated with protection against oxidative stress are preferably selected from the group consisting of BIRC3, CCL2, GZMB, SOD2, CCL5 and MMP1, and in particular selected from the group consisting of BIRC3, CCL2, GZMB and SOD2. More specifically, cell division is associated with decreased BCL2L1 expression, decreased NOX1 expression, increased SERPINE2 expression, increased BCL2 expression, increased CSF2 expression, increased CCND2 expression, and/or decreased SKP2 expression, as determined by differential expression analysis. Apoptosis is associated with increased CYP27B1 expression, increased IGHG1 expression and/or increased IGHE expression, as determined by differential expression analysis. Protection against oxidative stress is associated with increase BIRC3 expression, increased CCL2 expression, increased CCL5 expression, increased SOD2 expression, increased GZMB expression and increased MMP1 expression, as determined by differential expression analysis. An altered (e.g. increased) expression as determined by differential expression analysis as referred to herein means a change (e.g. increase) of an expression level of a FATG in a cell, whose respective cellular function is active (e.g. active cell division), as compared to an expression level of the FATG in a corresponding "non-activated" cell (e.g. inactive cell division).

According to a preferred embodiment, the cellular function is determined based on evaluating a mathematical model, in particular a calibrated mathematical model, relating the expression level of the at least one NFkB target gene (FATG) to the cellular function. Input to this model can be the expression level in a form provided by gene array analysis (a.u., log ratio, and the like), which are optionally normalized and/or corrected. Accordingly, it is an embodiment of the present invention that the expression level of the at least one NFkB target gene can be also an expression level normalized by and/or corrected for one or more of the following: (i) activity of the NFkB cellular signaling pathway in the cell sample; (ii) baseline expression level of the respective NFkB target gene(s); and/or (ii) average expression levels of the respective NFkB target gene(s) in a group of samples comprising cells with an active NFkB pathway. The baseline expression level is also referred to herein as reference expression level and denotes an expression level of the FATG in a cell, whose respective cellular function is inactive and/or whose NFkB signaling pathway is substantially inactive.

To increase reliability, expression levels of two or more FATGs may be determined for each cellular function and/or be input for the model. In such case, a function-associated expression average (herein also denoted as EA or FATG EA) may be calculated for each cellular function based on the expression levels of the two or more target genes associated with the respective cellular function. Moreover, the function-associated expression average may be based on normalized and/or corrected expression levels (as defined above). This means, for example, that the function-associated expression average may be based on averaged normalized expression levels (as determinable by differential expression analysis) of target genes associated with a particular cellular function, wherein the normalized expression level is defined by a ratio of the expression level of the FATG to the respective reference expression level.

Further, FATGs may be weighted according to their relevance; very informative FATGs may receive more weight than less informative FATGs. Specifically, BCL2, BCL2L1, CCND2 and SKP2 have been found to be more informative for indicating active cell division and may thus receive more weight than CSF1, NOX1 and SERPINE2. BIRC3, CCL2, GZMB and SOD2 have been found to be more informative for indicating protection against oxidative stress and may thus receive more weight than CCL5 and MMP1.

The determining of the cellular function may involve discriminating between the (i.e. a first) cellular function and at least one further (i.e. a second, etc.) cellular function based on expression levels of target genes associated with the respective cellular functions. For example, for each of two or more cellular functions, expression level of at least one FATG is determined. The expression levels may be normalized and/or corrected expression levels (as defined above). Further, two or more FATGs are preferably tested and the resulting EA determined (as described above) for each cellular function. As a result of the discrimination the method (in particular the model) may identify the predominant cellular function in the analyzed cell sample. Moreover, the method (model) may provide a ranking. The ranking may indicate which of the evaluated cellular functions is the most predominant and/or is the least predominant and/or is likely to be active and/or is likely to be the most active and/or is likely to be the least active and/or is likely to be inactive and/or the like.

According to a preferred embodiment, the cell sample is derived from a tissue, cells, blood and/or a body fluid such as a bronchial aspirate, bone marrow aspirate or a sample drawn from a body cavity of a subject, a cell line, a cell culture or a tissue culture. The cell sample may be a sample derived from a subject. The cell sample may be a sample derived from the subject and cultivated in vitro in the lab (e.g., for regenerative medicine purposes). The sample can be, e.g., a sample obtained from a cancer lesion, or from a lesion suspected for cancer, or from a metastatic tumor, or from a body cavity in which fluid is present which is contaminated with cancer cells (e.g., pleural or abdominal cavity or bladder cavity), or from other body fluids containing cancer cells, and so forth, preferably via a biopsy procedure or other sample extraction procedure. The cells of which a sample is extracted may also be tumorous cells from hematologic malignancies (such as leukemia or lymphoma). In some cases, the cell sample may also be circulating tumor cells, that is, tumor cells that have entered the bloodstream and may be extracted using suitable isolation techniques, e.g., apheresis or conventional venous blood withdrawal. Aside from blood, the body fluid of which a sample is extracted may be urine, gastrointestinal contents, or an extravasate. The term "cell sample", as used herein, also encompasses the case where tissue and/or cells and/or body fluid of the subject have been taken from the subject and, e.g., have been put on a microscope slide, and where for performing the claimed method a portion of this sample is extracted, e.g., by means of Laser Capture Microdissection (LCM), or by scraping off the cells of interest from the slide, or by fluorescence-activated cell sorting techniques. The cells or tissue can also be from normal, non-malignant tissue, or from diseased tissue other than cancer.

The term "subject", as used herein, refers to any living being. In some embodiments, the subject is an animal, preferably a mammal. In certain embodiments, the subject is a human being, preferably a medical subject, in particular a diseased subject such as a subject having an immune-related condition and/or cancer, or a subject having a risk of developing, or assumed to have, an immune-related condition and/or cancer. Alternatively, the cell sample may be a sample derived from a healthy subject.

According to a preferred embodiment, the cells contained in the cell sample are selected from the group consisting of immune cells and cancer cells.

As described above, an active NFkB cellular signaling pathway is determinable by pathway analysis as described elsewhere. Pathway analysis enables quantitative measurement of signal transduction pathway activity in epithelial cells, based on inferring activity of a signal transduction pathway from measurements of mRNA levels of the well-validated direct target genes of the transcription factor associated with the respective signaling pathway (see for example W Verhaegh, et al., Selection of personalized patient therapy through the use of knowledge-based computational models that identify tumor-driving signal transduction pathways. Cancer research 2014;74(11):2936-45; W Verhaegh, A van de Stolpe. Knowledge-based computational models. Oncotarget 2014;5(14):5196).

In accordance with pathway analysis, the activity of the NFkB cellular signaling pathway in the cells of the cell sample to be selected is inferred or inferable by a method comprising: receiving expression levels of one or more and preferably six or more target genes of the NFkB cellular signaling pathway, determining an level of an NFkB transcription factor (TF) element, the NFkB TF element controlling transcription of the one or more and preferably six or more target genes of the NFkB cellular signaling pathway, the determining being based at least in part on evaluating a mathematical model relating expression levels of the one or more and preferably six or more target genes of the NFkB cellular signaling pathway to the level of the NFkB TF element, wherein the one or more and preferably six or more target genes are selected from the group consisting of: BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TNIP 1, TRAF 1 and VCAM 1; inferring the activity of the NFkB cellular signaling pathway based on the determined level of the NFkB TF element in the sample, wherein the inferring is performed by a digital processing device using the mathematical model.

With respect to explanations and/or definitions of terms, particular embodiments including specific models, further target genes and/or preferred combinations of target genes as well as the respective sequence information, reference is made to WO 2017/029215 A1, which is incorporated herein by reference in its entirety and in particular in respect of the aformentioned details.

In accordance with another disclosed aspect, an apparatus comprises a digital processor configured to perform a method according to the present invention as described herein.

In accordance with another disclosed aspect, a non-transitory storage medium stores instructions that are executable by a digital processing device to perform a method according to the present invention as described herein. The non-transitory storage medium may be a computer-readable storage medium, such as a hard drive or other magnetic storage medium, an optical disk or other optical storage medium, a random access memory (RAM), read only memory (ROM), flash memory, or other electronic storage medium, a network server, or so forth. The digital processing device may be a handheld device (e.g., a personal data assistant or smartphone), a notebook computer, a desktop computer, a tablet computer or device, a remote network server, or so forth.

In accordance with another disclosed aspect, a computer program comprises program code means for causing a digital processing device to perform a method according to the present invention as described herein, when the computer program is run on the digital processing device. The digital processing device may be a handheld device (e.g., a personal data assistant or smartphone), a notebook computer, a desktop computer, a tablet computer or device, a remote network server, or so forth.

In accordance with another disclosed aspect, a kit for determining at least one cellular function of an active NFkB cellular signaling pathway comprises components for determining the expression levels of at least one NFkB target gene associated with a cellular function. The kit may be used in a method of diagnosis of a mammal, the diagnosis being based on determining at least one cellular function of an active NFkB cellular signaling pathway in the mammal. Optionally, the kit further comprises the herein disclosed apparatus, the herein disclosed non-transitory storage medium, or the herein disclosed computer program. Preferably, the kit comprises components for determining expression levels of one, a combination of two or more, or all of the following FATGs: BCL2L1, BCL2, CCND2, SKP2, NOX1, SERPINE2, CSF2, CYP27B1, IGHG1, IGHE, BIRC3, CCL2, SOD2, GZMB, CCL5 and MMP1. More preferably, the kit comprises components for determining expression levels of one, a combination of two or more, or all of the following FATGs: BCL2L1, BCL2, CCND2, SKP2, CYP27B1, IGHG1, IGHE, BIRC3, CCL2, SOD2 and GZMB. A combination of FATGs as used herein refers to any permutation of 2, 3, 4, 5, 6, 7, 8, 9, 10, etc. FATGs. The kit may comprises one or more components or means for measuring (in particular quantifying) the expression levels of the target genes selected from the group consisting of: a DNA array chip, an oligonucleotide array chip, a protein array chip, an antibody, a plurality of probes, for example, labeled probes, a set of RNA reverse-transcriptase sequencing components, and/or RNA or DNA, including cDNA, amplification primers. In a preferred embodiment, the kit is selected from the group consisting of qPCR, multiple qPCR, multiplexed qPCR, ddPCR, RNAseq, RNA expression array and mass spectrometry. In an embodiment, the kit includes a set of labeled probes directed to a portion of an mRNA or cDNA sequence of the target genes as described herein. In an embodiment, the kit includes a set of primers and probes directed to a portion of an mRNA or cDNA sequence of the target genes. In an embodiment, the labeled probes are contained in a standardized 96-well plate. In an embodiment, the kit further includes primers or probes directed to a set of reference genes. Such reference genes can be, for example, constitutively expressed genes useful in normalizing or standardizing expression levels of the target gene expression levels described herein.

In some embodiments, the kit is not a whole genome microarray. For example, the kit may at most be suited to determine expression levels of less than 1,000,000, less than 100,000 or less than 10,000 of target genes. For example, the kit of the present invention may include components for determining expression levels of not more than 1000 target genes, not more than 700 target genes, not more than 500 target genes, not more than 200 target genes, not more than 100 target genes, in addition to the components required for the specific target genes disclosed herein.

Another aspect of the present invention is a method of diagnosis or prognosis of a mammal, the diagnosis being based on determining at least one cellular function of an active NFkB cellular signaling pathway in the mammal.

Further advantages will be apparent to those of ordinary skill in the art upon reading and understanding the attached figures, the following description and, in particular, upon reading the detailed examples provided herein below.

This application describes several preferred embodiments. Modifications and alterations may occur to others upon reading and understanding the preceding detailed description. It is intended that the application is construed as including all such modifications and alterations insofar as they come within the scope of the appended claims or the equivalents thereof.

Other variations to the disclosed embodiments can be understood and effected by those skilled in the art in practicing the claimed invention, from a study of the drawings, the disclosure, and the appended claims.

It shall be understood that the herein disclosed methods, the herein disclosed apparatus, the herein disclosed non-transitory storage medium, the herein disclosed computer program, and the herein disclosed kits have similar and/or identical preferred embodiments, in particular, as defined in the dependent claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage. Calculations performed by one or several units or devices can be performed by any other number of units or devices.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:

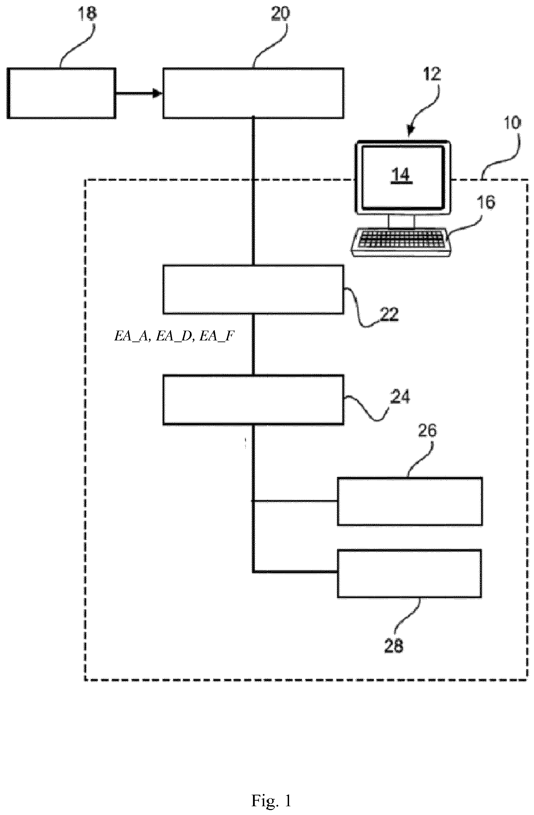

Fig. 1 schematically shows a CDS system, which provides clinical decision support based on the determined cellular function of an active NFkB cellular signaling pathway in a cell sample, as disclosed herein.

### DETAILED DESCRIPTION OF EMBODIMENTS

The following embodiments merely illustrate particularly preferred methods and selected aspects in connection therewith. The teaching provided therein may be used for constructing several tests and/or kits. The following examples are not to be construed as limiting the scope of the present invention.

The present invention relates to means and methods that can identify a cellular function of an active NFkB cellular signaling pathway. Knowing the cellular function may assist in selection of a therapy, in particular cancer therapy. The present invention focuses on the NFκB (herein also referred to as NFkB) pathway. The NFκB pathway is one of the most important signal transduction pathways in the immune response, and also plays important roles in other cells, notably cancer cells.

The present invention for the first time facilitates identification of the (predominant) cellular function of an active NFκB signaling pathway. Activity of an NFκB signaling pathway can be determined by a computational model based on measurement of the transcription factor target gene levels, where target genes were carefully selected from the existing literature as described in WO 2017/029215 A1, which is incorporated herein by reference in its entirety. As described in the introduction, activity of the NFkB pathway does however not provide information about the functional role, which the NFkB pathway exerts in a cell. The present inventors found that certain NFkB target genes are differentially expressed between different cellular function of an active NFkB pathway. These target genes are denoted as cellular function-associated NFkB target genes (FATGs).

### Identification of cellular function-associated NFkB target genes (FATGs)

The NFkB pathway regulates multiple cellular functions. A method is described by which important cellular functions can be determined of an active NFkB pathway: cell division, apoptosis and oxidative stress.

Affymetrix U133Plus2.0 mRNA expression microarray datasets from the GEO database (https://www.ncbi.nlm.nih.gov/gds/) were used to identify NFkB active samples that were associated with a "ground truth" NFkB cellular function. Unless otherwise indicated, expression levels are given in the following as differential expression levels (log odd) gathered from the corresponding raw data received from the Affymetrix microarray datasets.

For each NFkB activity-associated cellular function one prototypic dataset was defined which was used to discover cellular function-associated NFkB target genes (cf. Table 1).

**Table 1: Definition of a prototypic dataset for each NFkB functional state. The prototypic dataset was used as target gene discovery and calibration dataset.**

| **Cellular Function** | **Comparison (active vs. inactive cellular function)** | **Calibration dataset** |
|---|---|---|
| Cell division | PBMC: 24hr vs 0hr after IL2 withdrawal | GSE7345 |
| | NK: 24hr vs 0hr IL2 stimulation | GSE8059 |
| Cell Apoptosis | MM1.S cells: beta-catenin knockdown vs control | GSE17385 |
| Protection against oxidative stress | Basal breast cancer with SOD above threshold vs normal | GSE45827 |

Next to these calibration 'discovery' datasets, independent Affymetrix validation datasets were also selected, also containing a ground truth per sample-data, and these were used to validate the cellular function-associated NFkB target genes (FATG). Subsequently for each sample in the datasets NFkB pathway activity analysis (NFkB activity in the sample data was measured in a quantitative manner using the previously described NFkB computational model in WO 2017/029215 A1) was performed and the functional status of each analyzed cell sample was listed according to the 'ground truth' provided by the authors of the corresponding publication or the annotation provided in GEO.

Subsequently, to discover FATGs for each cellular function of NFkB, in the discovery datasets (cf. Table 1), NFkB active samples with an annotated "ground truth" with respect to the NFkB cellular function, individual NFkB target gene (comprising the target genes of the activity model plus extended list of target genes) expression levels were compared between sample groups with the different designated functions, to discover NFkB target genes that were associated with one of the mentioned functions.

As NFkB target genes to investigate with respect to differential expression in the comparison between function-associated datasets, the original set of target genes as disclosed in WO 2017/029215 A1 was used complemented with additional target genes of which sufficient literature evidence was available to provide evidence that the target gene was a direct NFkB target gene:
A. Target genes used in original pathway activity model (WO 2017/029215 A1): BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TNIP1, TRAF1 and VCAM1
B. Additionally selected NFKB target genes from literature: ABCA1, ABCB4, ABCC6, ABCG8, ADH1A, ADORA1, ADORA2A, AFP, AGER, AGT, ALOX12, APOBEC2, APOC3, APOD, APOE, AQP4, AR, ARFRP1, ASPH, ASS1, ATP1A2, B2M, BACE1, BAX, BCL2, BCL2L11, BCL3, BDKRB1, BDNF, BLNK, BMP2, BMP4, BNIP3, BRCA2, BTK, C4BPA, C69, CASP4, CCL1, CCL19, CCL23, CCND1, CCND2, CCR5, CCR7, CD209, CD274, CD38, CD3G, CD40, CD40LG, CD44, CD48, CD80, CD83, CD86, CDX1, CEBPD, CFB, CHI3L1, CIDEA, COL1A2, CR2, CREB3, CRP, CSF2, CSF3, CTSB, CXCL5, CXCL9, CYP19A1, CYP27B1, CYP7B1, DIO2, DNASE1L2, EBI3, EDN1, EGFR, ELF3, ENG, EPHA1, EPO, ERBB2, F3, F8, FABP6, FAM148A, FAS, FASLG, FCER2, FCGRT, FGF8, FN1, FSTL3, FTH1, G6PC, GADD45B, GATA,3 GBP1, GCLC, GCLM, GNAI2, GNB2L1, GNRH2, GZMB, HAMP, HAS1, HBE1, HBZ, HIF1A, HLA-G, HOXA9, HSD11B2, HSP90AA1, IER3, IFNB1, IFNG, IGFBP2, IGHE, IGHG1, IL10, IL11, IL12A, IL12B, IL13, IL17, ILIA, IL1RN, IL27, IL2RA, IL8RA, IL8RB, IL9, IRF2, IRF4, IRF7, JUNB, KCNK5, KCNN2, KISS1, KITLG, KLK3, KLRA1, KRT1,5 KRT3, KRT5, KRT6B, LAMB2, LBP, LCN2, LEF1, LGALS3, LIPG, LTA, LTB, LYZ, MADCAM1, MBP, MMP1, MMP3, MTHFR, MUC2, MYB, MYC, MYLK, MYOZ1, NCAM, NFKB1, NFKBIZ, NLRP2, NOD2, NOS1, NOS2A, NOX1, NPY1R, NQO1, NR4A2, NRG1, NUAK2, OLR1, OPN1SW, OPRD1, OPRM1, ORM1, OXTR, PAFAH2, PDGFB, PDYN, PENK, PGLYRP1, PGR, PIGF, PIGR, PIK3CA, PLAU, PLCD1, PLK3, POMC, PRF1, PRKACA, PRKCD, PRL, PSMB9, PSME1, PSME2, PTEN, PTGDS, PTHLH, PTPN1, PTX3, RAG1, RAG2, RBBP4, REL, RELB, S100A4, S100A6, SAA1, SAT1, SCNN1A, SDC4, SELP, SELS, SERPINA1, SERPINA3, SERPINB1, SERPINE1, SERPINE2, SH3BGRL, SKALP, SKP2 SLC11A2, SLC16A1, SLC3A2, SLC6A6, SNAI1, SOD1, SOD2, SOX9, SPI,1 SPP1, ST6GAL1, ST8SIA1, TACR1, TAP1, TAPBP, TCRB, TERT, TFEC, TFF3, TGM1, TGM2, TICAM1, TLR2, TLR9, TNC, TNFAIP3, TNFRSF4, TNFRSF9, TNFSF10, TNFSF13B, TNFSF15, TNIP3, TP53, TREM1, TRPC1, TWIST1, UPK1B, UPP1, VEGFC, VIM, WT1 and YY1.

Genes that were associated with a function, based on the differential expression study on public datasets, were preferred for the Function-Associated Target Gene (FATG) list (cf. Table 2).

**Table 2: Discovery of Function-Associated Target Gene (FATG). The function was defined based on discovery of genes in the respective datasets.**

| **Function** | Very informative (I+) genes | Informative (I) genes |
|---|---|---|
| Cell division | BCL2, BCL2L1, CCND2, SKP2 | CSF1, NOX1, SERPINE2 |
| Cell Apoptosis | CYP27B1, IGHE / IGHG1 (same probe sets; could not be separated) | |
| Protection oxidative stress | BIRC3, CCL2, GZMB, SOD2 | CCL5, MMP1 |

For each cellular function of NFkB between 2 and 15 NFkB target genes were defined as FATG. Genes were divided in two categories FATGs: (a) category "I+", meaning very informative with respect to the cellular function, and (b) category "I", meaning informative. These informative target genes were only included in the FATG list if differential expression results (associated with NFkB function in the discovery datasets compared) agreed with the known biological function. For some genes expression results were obtained with two or more probe sets. Differential expression results of the discovery datasets for the genes of the FATG list are displayed in Tables 3 to 5. Differential expression results of very informative and informative genes have respectively a bold and normal font.

**Table 3: Results from differential expression study of activated / non-activated samples to identify the FATG genes. Cellular Function: Cell Division. Cell division was activated by stimulation with IL2 and blocked by withdrawal of IL2. Expression levels of "active" cells have been normalized by the expression level of "inactive" cells (normalized expression levels).**

| **Discovery dataset** | GSE7345 | GSE8059 |
|---|---|---|
| **Comparison (active vs. inactive cell division)** | PBMC: 24 hr/ 0 hr after IL-2 withdrawal. | NK: 24 hr / 0 hr stimulation IL-2. |
| | Indicated is delta between 24 and 0 hours. | Indicated is delta between 24 and 0 hours. |
| BCL2L1 | **0.387961** | **2.237027** |
| BCL2L1 | **0.387961** | **1.205173** |
| NOX1 | 0.809677 | 2.798021 |
| SERPINE2 | 2.179479 | 1.738923 |
| BCL2 | **1.904978** | **3.203082** |
| BCL2 | **1.955548** | **2.197475** |
| CSF2 | 2.293724 | 2.276276 |
| CCND2 | **2.117936** | **1.403538** |
| CCND2 | **1.563048** | **1.671844** |
| SKP2 | **0.583479** | **0.898562** |

**Table 4: Results from differential expression study of activated / non-activated samples to identify the FATG genes (normalized expression levels). Cellular Function: Apoptosis.**

| **Discovery dataset** | GSE17385 |
|---|---|
| **Comparison** | MM1.S cells: beta-catenin knock-down /control |
| CYP27B1 | **2.411292** |
| IGHG1 | **2.003433** |
| IGHE | **2.003433** |

**Table 5: Results from differential expression study of activated / non-activated samples to identify the FATG genes (normalized expression levels). Cellular Function: Oxidative stress.**

| **Discovery dataset** | GSE45827 |
|---|---|
| **Comparison** | BC: Basal Sod high / normal |
| BIRC3 | **2.867982** |
| CCL2 | **3.332631** |
| CCL5 | 1.927742 |
| CCL5 | 2.541469 |
| CCL5 | 1.701361 |
| SOD2 | **2.786796** |
| SOD2 | **3.200371** |
| GZMB | **3.456095** |
| MMP1 | 4.539692 |

It is conceivable that following collection of immune cell data in the future, using this method, the FATG list can be further improved based on data analysis, and target gene combinations selected that further improve the function of the here described NFkB pathway-associated cellular function analysis.

The FATGs identified above were validated using a ranking model as defined below on validation datasets.

### Computational model for validation of FATGs

### Type 1 model. Function-associated expression average (EA) ranking model.

According to the type 1 model, a cellular function of an active NFkB pathway in a cell is predicted based on measured mRNA levels of FATGs determined in an individual sample.
As an example the mRNA levels are measured on an Affymetrix U133Plus2.0 microarray. First the NFkB activity level is determined using the method disclosed in WO 2017/029215 A1. Subsequently for each NFkB function (cell division, differentiation, chemotaxis, adhesion, apoptosis, oxidative stress) expression levels of the respective FATG genes are taken and amplified by a factor 1 (gene from the FATG category "informative", called "I") or 2 (gene from the FATG category " very informative", called "I+"); subsequently values are added and divided by: the number of informative FATG genes+the number of very informative FATG genes (that have been counted twice). This delivers the expression average, EA, which allows identification of the function with the highest EA. This is performed for all functions.

Subsequently, expression averages (EA) of all these calculations are ranked from high to low, and function(s) of the NFkB pathway can be read from high to low. When the NFkB pathway is active, it can induce either one or multiple cellular functions. Associated EA scores indicate not only which function is the dominant one, but also which other functions were induced by activity of the NFkB pathway.

The EA can be calculated for each NFkB function as follows: $EA = \left[1\times \left(FATG-I,1+FATG-I,2+…FATG-I,N\right)+2\times \left(FATG-I+,1+FATG-I+,2+…FATG-I,N\right)\right] / \left[1\times \left(number of FATG-I genes\right)+2\times \left(number of FATG-I+genes\right)\right] ,$ wherein FATG-I denote expression level of informative gene and 1, 2, ..., N are informative genes for the respective function, wherein FATG-I+ denote expression level of very informative gene and 1, 2, ..., N are very informative genes for the respective function.

NFkB functions can be named according to A=chemotaxis, B = antigen presentation, C= cell adhesion, D = cell differentiation, F= cell apoptosis, F=oxidative stress; EA scores for all functions (A-F) are ranked, associated NFkB functions for the investigated sample are ranked from highest EA score to lowest EA score.

An oxidative stress function is exemplarily calculated as follows:
EA= [2xBIRC3 + 2xCCL2 + (CCL5(P1) + CCL5(P2) + CCL5(P3))/3 + 2x(SOD2(P1) + SOD2(P2))/2 + 2xGZMB + MMP1] / 10, wherein P1, P2 and P3 refer to three different probe sets used for determining the (normalized) expression level of the same FATG (CCL5).

The above detailed ranking model has been used to analyze all individual samples of all validation datasets for each of the cellular NFkB functions oxidative stress, apoptosis, and cell division.

The following Tables 6 to 10 show validation results on public Affymetrix U133Plus2.0 microarray datasets. The GSE number indicates the GEO dataset, GSM numbers indicate individual samples on which an Affymetrix U133 Plus2.0 was performed. EA scores for each function ("CD"=cell division, "APOP"=apoptosis, "OS"=oxidative stress) are indicated per sample (column: "score"), as well as function rank (column: "rank"). Functions are ranked 1 to 3, where 1 has highest EA score and indicates the most dominant cellular function of the active NFkB pathway, and 3 the lowest EA score, indicating the least dominant cellular function. NFkB pathway activity for each individual analyzed sample, ranked from low to high activity score, is given as log 2odds score in column "NFkB log2odd".

Tables 6 to 10 show validation results using dataset GSE45827, with basal-type and luminal A-type breast cancer samples (Gruosso T, Mieulet V, Cardon M, Bourachot B et al., EMBO Mol Med 2016 May;8(5):527-49). In this study the oxidative stress state was measured in analyzed cancer samples using immunohistochemistry staining developed by the authors. They show that in Lumina A breast cancer oxidative stress is lowest, while highest in basal type breast cancer samples. This was confirmed by the inventor's analyses (van Ooijen et al., The American Journal of Pathology, 2018, vol. 188, no. 9, p. 1956-1972).

For each cellular function (CD, APOP and OS) a weighted sum of expression levels ("WSE") is given. The sum of expression levels of the FATGs disclosed herein for the respective cellular function was weighted by the informativeness of the respective cellular function, i.e. 1 x (FATG-I,1 + FATG-I,2 + ...FATG-I,N) + 2x(FATG-I+,1 + FATG-I+,2 + ...FATG-I,N), as described herein. In case a FATG is represented by 2 or more probesets, the average of the number of contributing probe-sets is taken. The score was then calculated by dividing this weighted sum of expression levels by the weighted number of FATGs, i.e. 1x (number of FATG-I genes) + 2x (number of FATG-I+ genes), as described herein.

**Table 6: Validation results on GEO dataset GSE45827 with basal-type breast cancer samples, NFkB pathway active (50 % samples with highest NFkB).**

| **Array ID GSM...** | **CD** | | | **APOP** | | | **OS** | | | **NFkB** |
|---|---|---|---|---|---|---|---|---|---|---|
| | **WSE** | **score** | **rank** | **WSE** | **score** | **rank** | **WSE** | **score** | **rank** | **log2odd** |
| 1116148 | 75.51 | 6.86 | 3 | 37.88 | 9.47 | 2 | 96.88 | 9.69 | 1 | 32.82 |
| 1116102 | 69.85 | 6.35 | 3 | 29.45 | 7.36 | 2 | 84.44 | 8.44 | 1 | 31.67 |
| 1116136 | 77.20 | 7.02 | 3 | 36.46 | 9.12 | 2 | 93.55 | 9.35 | 1 | 31.45 |
| 1116124 | 72.94 | 6.63 | 3 | 38.36 | 9.59 | 2 | 98.70 | 9.87 | 1 | 28.87 |
| 1116145 | 68.90 | 6.26 | 3 | 37.39 | 9.35 | 2 | 90.53 | 9.05 | 1 | 27.01 |
| 1116111 | 73.15 | 6.65 | 3 | 40.43 | 10.11 | 1 | 86.68 | 8.67 | 2 | 26.66 |
| 1116131 | 70.48 | 6.41 | 3 | 37.79 | 9.45 | 2 | 87.05 | 8.70 | 1 | 26.57 |
| 1116091 | 74.16 | 6.74 | 3 | 42.11 | 10.53 | 1 | 92.89 | 9.29 | 2 | 25.31 |
| 1116115 | 78.10 | 7.10 | 3 | 38.94 | 9.73 | 2 | 91.11 | 9.11 | 1 | 25.28 |
| 1116109 | 78.97 | 7.18 | 3 | 40.18 | 10.04 | 1 | 86.37 | 8.64 | 2 | 24.54 |
| 1116101 | 74.36 | 6.76 | 3 | 40.71 | 10.18 | 1 | 90.39 | 9.04 | 2 | 24.32 |
| 1116147 | 69.45 | 6.31 | 3 | 42.05 | 10.51 | 1 | 92.04 | 9.20 | 2 | 23.91 |
| 1116143 | 67.05 | 6.10 | 3 | 38.05 | 9.51 | 1 | 86.12 | 8.61 | 2 | 23.53 |
| 1116090 | 72.59 | 6.60 | 3 | 40.62 | 10.15 | 1 | 88.35 | 8.83 | 2 | 22.79 |
| 1116094 | 74.31 | 6.76 | 3 | 43.87 | 10.97 | 1 | 90.35 | 9.04 | 2 | 22.60 |
| 1116084 | 74.42 | 6.77 | 3 | 38.68 | 9.67 | 1 | 81.83 | 8.18 | 2 | 21.38 |
| 1116112 | 70.41 | 6.40 | 3 | 38.40 | 9.60 | 1 | 77.31 | 7.73 | 2 | 19.26 |
| 1116085 | 72.36 | 6.58 | 3 | 33.46 | 8.36 | 2 | 83.79 | 8.38 | 1 | 19.04 |
| 1116135 | 69.32 | 6.30 | 3 | 38.23 | 9.56 | 1 | 82.40 | 8.24 | 2 | 18.92 |
| 1116144 | 71.83 | 6.53 | 3 | 41.49 | 10.37 | 1 | 82.60 | 8.26 | 2 | 18.30 |
| **average** | 72.77 | 6.62 | 3 | 38.73 | 9.68 | 1.4 | 88.17 | 8.82 | 1.6 | |

It can be seen from Table 6 that oxidative stress is prominent (n=8 out of 20 (40%) with score 1 for oxidative stress function) in the samples with NFkB activity (left figure), and apoptosis function ranking as first (1) in 12 out of 20 samples. Since cell division function in general scores 3, it is not the most important function of the NFkB pathway in this basal type breast cancer, which is presumably driven by other cell division pathways, like the PI3K and MAPK pathways.

**Table 7: Validation results on GEO dataset GSE45827 with luminal A-type breast cancer samples, NFkB pathway active (50 % samples with highest NFkB).**

| **Array ID GSM...** | **CD** | | | **APOP** | | | **OS** | | | **NFkB** |
|---|---|---|---|---|---|---|---|---|---|---|
| | **WSE** | **score** | **rank** | **WSE** | **score** | **rank** | **WSE** | **score** | **rank** | **log2odd** |
| 1116190 | 75.43 | 6.86 | 3 | 41.73 | 10.43 | 1 | 75.85 | 7.58 | 2 | 21.93 |
| 1116222 | 71.60 | 6.51 | 3 | 43.78 | 10.95 | 1 | 72.35 | 7.24 | 2 | 21.13 |
| 1116223 | 70.46 | 6.41 | 3 | 41.24 | 10.31 | 1 | 68.32 | 6.83 | 2 | 18.84 |
| 1116191 | 71.35 | 6.49 | 3 | 36.52 | 9.13 | 1 | 72.07 | 7.21 | 2 | 18.81 |
| 1116235 | 73.06 | 6.64 | 2 | 39.75 | 9.94 | 1 | 63.71 | 6.37 | 3 | 18.58 |
| 1116204 | 71.65 | 6.51 | 3 | 42.51 | 10.63 | 1 | 68.59 | 6.86 | 2 | 14.44 |
| 1116209 | 70.77 | 6.43 | 2 | 41.24 | 10.31 | 1 | 63.68 | 6.37 | 3 | 13.64 |
| 1116203 | 71.26 | 6.48 | 2 | 37.67 | 9.42 | 1 | 60.81 | 6.08 | 3 | 13.11 |
| 1116181 | 73.45 | 6.68 | 3 | 38.36 | 9.59 | 1 | 70.66 | 7.07 | 2 | 12.68 |
| 1116228 | 73.29 | 6.66 | 3 | 40.48 | 10.12 | 1 | 68.63 | 6.86 | 2 | 12.43 |
| 1116194 | 72.68 | 6.61 | 3 | 41.57 | 10.39 | 1 | 68.74 | 6.87 | 2 | 11.68 |
| 1116200 | 72.15 | 6.56 | 2 | 41.23 | 10.31 | 1 | 58.96 | 5.90 | 3 | 10.10 |
| 1116205 | 69.53 | 6.32 | 2 | 41.47 | 10.37 | 1 | 61.87 | 6.19 | 3 | 10.04 |
| 1116231 | 72.22 | 6.57 | 2 | 35.99 | 9.00 | 1 | 61.76 | 6.18 | 3 | 9.08 |
| **average** | 72.06 | 6.55 | 2.57 | 40.25 | 10.06 | 1 | 66.86 | 6.69 | 2.43 | |

As can be gathered from Table 7, none of the 14 samples (0%) scores oxidative stress as most prominent function of the active NFkB pathway, instead all 14 samples score apoptosis as prime function. A few of the samples have cell division function scored as (2), suggesting that the NFkB pathway may play a role in cell division in a subset of these tumors.

To provide additional proof for the correct measurement of the oxidative stress versus apoptosis function of the NFkB pathway, the cell division marker MKI67 was also measured in these samples, based on the expression levels measured on the Affymetrix microarrays. Results are shown of the four probe sets available on the Affymetrix microarray. Clearly, MKI67 levels are high in the basal like breast cancer and low in Luminal A, supporting that the apoptosis function was indeed prime in Luminal A, and the oxidative stress function without apoptosis important in many basal-like samples (cf. Table 8).

**Table 8: MKI67 levels in samples from dataset GSE45827.**

| **MKI67 probe set** | **Basal like breast cancer cells** | | | **Luminal A type breast cancer cells** | | |
|---|---|---|---|---|---|---|
| | **average** | **max** | **min** | **average** | **max** | **min** |
| MKI67_212020_s_at | 7.02 | 7.81 | 6.26 | 4.93 | 5.67 | 4.55 |
| MKI67_212021_s_at | 8.03 | 8.76 | 7.45 | 5.57 | 6.33 | 4.92 |
| MKI67_212022_s_at | 7.84 | 8.88 | 6.85 | 5.08 | 5.80 | 4.14 |
| MKI67_212023_s_at | 6.86 | 7.69 | 6.05 | 4.70 | 5.66 | 4.18 |

In Table 9, the basal like samples with lowest NFkB pathway activity (assuming a very inactive NFkB pathway; notwithstanding, on the relative NFkB pathway activity score scale as described herein, these samples are considered to be active) are shown. The associated MKI67 expression levels are shown in Table 10. This shows that the cell division in these samples is independent of NFkB activity and caused by another signaling pathway. MKI67 in basals with low NFkB (indicating an inactive or minimally active pathway) is on average even higher than in basals with high NFkB, strengthening the assumption that cell division in basals is unrelated to NFkB activity.

**Table 9: Validation results on GEO dataset GSE45827 with Basal breast cancer samples with low NFkB log2odd values, NFkB pathway relatively inactive (50% samples with lowest NFkB).**

| **Array ID GSM...** | **NFkB log2odd** |
|---|---|
| 1116106 | 17.02 |
| 1116139 | 16.22 |
| 1116113 | 14.96 |
| 1116150 | 14.62 |
| 1116120 | 14.59 |
| 1116108 | 13.71 |
| 1116110 | 12.80 |
| 1116130 | 12.51 |
| 1116142 | 12.40 |
| 1116099 | 12.23 |
| 1116119 | 11.21 |
| 1116128 | 9.98 |
| 1116114 | 9.62 |
| 1116092 | 7.84 |
| 1116125 | 6.71 |
| 1116146 | 3.29 |
| 1116087 | 2.78 |
| 1116118 | -2.71 |
| 1116138 | -5.43 |
| 1116093 | -7.52 |
| 1116149 | -7.72 |

**Table 10: MKI67 expression levels associated with subjects with Basal breast cancer with low NFkB log2odd values.**

| | **MKI67_212020_s_at** | **MKI67_212021_s_at** | **MKI67_212022_s_at** | **MKI67_212023_s_at** |
|---|---|---|---|---|
| **average** | 7.31 | 8.39 | 8.23 | 7.12 |
| **max** | 8.74 | 9.55 | 9.92 | 8.84 |
| **min** | 6.08 | 6.79 | 6.24 | 6.02 |

Table 11 shows validation results for a second basal set (GEO dataset GSE76275). The associated MKI67 expression levels are shown in Table 12. Score, ranking and MKI-67 profile are very similar to the results in regard of GSE45827. It was shown that 11 out of 27 samples (41%) score oxidative stress as the most important function of the NFkB pathway, confirming the results shown in Table 6. MKI67 results support the results of oxidative stress associated with high cell division rate.

**Table 11: Validation results on dataset GSE76275 containing Basal-like breast cancer samples with an active NFkB pathway.**

| **Array ID GSM...** | **CD** | | | **APOP** | | | **OS** | | | **NFkB** |
|---|---|---|---|---|---|---|---|---|---|---|
| | **WSE** | **score** | **rank** | **WSE** | **score** | **rank** | **WSE** | **score** | **rank** | **log2odd** |
| 1974604 | 77.64 | 7.06 | 3 | 41.00 | 10.25 | 2 | 108.08 | 10.81 | 1 | 31.93 |
| 1974614 | 72.75 | 6.61 | 3 | 37.33 | 9.33 | 2 | 104.02 | 10.40 | 1 | 29.87 |
| 1974626 | 73.99 | 6.73 | 3 | 41.49 | 10.37 | 1 | 91.67 | 9.17 | 2 | 29.76 |
| 1974593 | 71.49 | 6.50 | 3 | 43.28 | 10.82 | 2 | 110.50 | 11.05 | 1 | 28.62 |
| 1974567 | 77.13 | 7.01 | 3 | 36.96 | 9.24 | 2 | 105.20 | 10.52 | 1 | 27.85 |
| 1974738 | 76.85 | 6.99 | 3 | 41.27 | 10.32 | 1 | 99.08 | 9.91 | 2 | 26.90 |
| 1974592 | 77.02 | 7.00 | 3 | 42.16 | 10.54 | 1 | 99.04 | 9.90 | 2 | 25.95 |
| 1974724 | 70.85 | 6.44 | 3 | 34.58 | 8.65 | 2 | 88.17 | 8.82 | 1 | 24.74 |
| 1974721 | 75.84 | 6.89 | 3 | 38.03 | 9.51 | 2 | 107.91 | 10.79 | 1 | 24.65 |
| 1974747 | 78.53 | 7.14 | 3 | 36.47 | 9.12 | 2 | 98.06 | 9.81 | 1 | 24.12 |
| 1974710 | 75.47 | 6.86 | 3 | 40.00 | 10.00 | 1 | 89.16 | 8.92 | 2 | 23.43 |
| 1974628 | 71.43 | 6.49 | 3 | 42.73 | 10.68 | 1 | 95.79 | 9.58 | 2 | 22.85 |
| 1974605 | 84.04 | 7.64 | 3 | 30.81 | 7.70 | 2 | 103.49 | 10.35 | 1 | 22.34 |
| 1974696 | 73.78 | 6.71 | 3 | 38.82 | 9.71 | 2 | 102.24 | 10.22 | 1 | 21.88 |
| 1974659 | 73.12 | 6.65 | 3 | 37.78 | 9.44 | 1 | 93.35 | 9.34 | 2 | 20.87 |
| 1974595 | 76.05 | 6.91 | 3 | 42.96 | 10.74 | 1 | 94.48 | 9.45 | 2 | 20.84 |
| 1974744 | 73.86 | 6.71 | 3 | 39.77 | 9.94 | 1 | 93.89 | 9.39 | 2 | 20.33 |
| 1974732 | 79.58 | 7.23 | 3 | 39.58 | 9.89 | 1 | 96.10 | 9.61 | 2 | 20.31 |
| 1974591 | 74.07 | 6.73 | 3 | 39.27 | 9.82 | 1 | 95.77 | 9.58 | 2 | 20.23 |
| 1974692 | 73.58 | 6.69 | 3 | 41.95 | 10.49 | 1 | 90.44 | 9.04 | 2 | 19.44 |
| 1974718 | 76.59 | 6.96 | 3 | 38.41 | 9.60 | 1 | 93.19 | 9.32 | 2 | 19.11 |
| 1974749 | 73.89 | 6.72 | 3 | 34.86 | 8.71 | 2 | 91.65 | 9.16 | 1 | 17.76 |
| 1974670 | 75.83 | 6.89 | 3 | 37.23 | 9.31 | 1 | 84.65 | 8.46 | 2 | 17.07 |
| 1974691 | 71.51 | 6.50 | 3 | 42.07 | 10.52 | 1 | 88.96 | 8.90 | 2 | 16.94 |
| 1974664 | 72.90 | 6.63 | 3 | 34.76 | 8.69 | 2 | 92.40 | 9.24 | 1 | 16.86 |
| 1974712 | 75.77 | 6.89 | 3 | 40.20 | 10.05 | 1 | 88.07 | 8.81 | 2 | 16.71 |
| 1974679 | 70.66 | 6.42 | 3 | 35.87 | 8.97 | 1 | 82.54 | 8.25 | 2 | 16.29 |
| **average** | 74.97 | 6.82 | 3 | 38.88 | 9.72 | 1.41 | 95.85 | 9.58 | 1.59 | |

**Table 12: MKI67 expression levels associated with Basal-like breast cancer samples with an active NFkB pathway. Remark: MKI67 activity levels for all four probe sets were very low for sample GSM1974732; sample has been excluded from analysis for being an outlier.**

| | **MKI67_212020_s_at** | **MKI67_212021_s_at** | **MKI67_212022_s_at** | **MKI67_212023_s_at** |
|---|---|---|---|---|
| **average** | 6.96 | 8.18 | 7.64 | 7.09 |
| **max** | 9.33 | 9.20 | 9.35 | 8.93 |
| **min** | 5.62 | 6.48 | 6.18 | 5.53 |

Table 13 shows validation results for GSE43657 dataset SUM159. The associated MKI67 expression levels are shown in Table 14. The GSE43657 dataset SUM159 contains cell line sample of the inflammatory breast cancer cell line SUM159. As can be seen in both samples Oxidative stress is ranked as (1), and cell-division as (2). The MKI67 levels are corresponding and high. This indicates that in this cell line oxidative stress is strongly related to the cell division function of NFkB, and the apoptotic pathway which normally protects against the DNA damaging effects of oxidative stress, is not activated by the NFkB pathway. That is, in this dataset NFkB related oxidative stress is high, apoptosis is low.

**Table 13: Validation results on GSE43657 dataset SUM159. GSM1067679: SUM159 cell line; GSM1067680: SUM159 sphere. Spheres were obtained by culturing cells in 3D in a spheroid, which generally reduces cell growth, but due to local hypoxia is still considered to be more associated with oxidative stress.**

| **Array ID GSM...** | **CD** | | | **APOP** | | | **OS** | | | **NFkB** |
|---|---|---|---|---|---|---|---|---|---|---|
| | **WSE** | **score** | **rank** | **WSE** | **score** | **rank** | **WSE** | **score** | **rank** | **log2odd** |
| 1067679 | 76.19 | 6.93 | 2 | 25.35 | 6.34 | 3 | 74.84 | 7.48 | 1 | 7.82 |
| 1067680 | 79.54 | 7.23 | 2 | 25.46 | 6.36 | 3 | 77.03 | 7.70 | 1 | 10.94 |
| **average** | 77.86 | 7.08 | 2 | 25.40 | 6.35 | 3 | 75.94 | 7.59 | 1 | |

**Table 14: MKI67 expression levels associated with GSE43657 dataset SUM159. GSM1067679: SUM159 cell line; GSM1067680: SUM159 sphere.**

| **Array ID GSM...** | MKI67_212020_s_at | MKI67_212021_s_at | MKI67_212022_s_at | MKI67_212023_s_at |
|---|---|---|---|---|
| 1067679 | 7.68 | 9.03 | 9.36 | 7.90 |
| 1067680 | 6.56 | 8.03 | 8.05 | 7.00 |
| **average** | 7.12 | 8.53 | 8.70 | 7.45 |

In summary, it can be seen that the average of the EA scores in Basal like breast cancer group is similar to the average EA scores for the Lumina A type breast cancer group for cell division function; for apoptosis function the average score is higher in the Luminal A group, and for the oxidative stress function the average of this score is much higher in the basal like breast cancer group. These results are in good agreement with literature on this patient cohort (Gruosso et al.; van Ooijen et al.; *supra*) providing additional support for the model to be able to correctly identify NFkB pathway -associated functions.

The validation results provide clear evidence that by using certain FATGs a distinction can be made between three important functional states of an active NFkB pathway, that is, oxidative stress, apoptosis, and cell division. An active NFkB pathway can induce transcription of genes like SOD2 which are needed to protect the DNA against the damaging effects of oxidative stress, which is called the "oxidative stress" function. In cancer, this oxidative stress is in general induced by rapid cell division induced by a signaling pathway, which controls cell division. This was clearly observed in the basal like breast cancer samples that were analyzed, and in which MKI67 as cell division marker was high.

That NFkB was not the pathway causing the cell division (ranked as lowest NFkB function, i.e. rank 3) could be confirmed by the fact that MKI67 was just as high in the basal like breast cancer samples with a low NFkB pathway activity; the observed cell division was independent of NFkB pathway activity.

The NFkB pathway can also direct the cell towards apoptosis, to prevent cell division of cells, whose DNA has been damaged by oxidative stress. In that case the pathway is protecting the tumor against accumulation of more mutations which might promote cancer progression. This situation was observed in the slow growing Luminal A breast cancer, further supported by a low MKI67 expression level. This low MKI67 level also confirms correctness of the determined rank (rank 3) for the cell division function of the NFkB pathway. Finally, only in the inflammatory breast cancer, dominant NFkB functions were oxidative stress with cell division. This is in line with a general bad prognosis of this tumor type and the role of inflammation (the NFkB pathway being the inflammatory pathway) therein.

All results provide firm evidence that cellular function such as oxidative stress, apoptosis and cell division of an active NFkB pathway can be correctly ranked and defined based on FATGs, for example by using an FATG EA function ranking for NFkB pathway activity as disclosed herein.

While the above scoring/ranking methodology (type 1 model) is advantageous in terms of its simplicity and the fact to no information is required from other samples, the present invention shall not be understood to be restricted to specific methodologies but is generally applicable. Alternative methodologies are presented in the following. Notably, the ranking following calculation of EA according to the various models may be identical for all presented scoring models.

### Type 2-model. Normalization using NFkB pathway activity score.

According to the type 2-model, the FATG EA score is determined as described above for the type 1-model and then corrected (normalized) by the NFkB pathway activity (score): EA/NFkB pathway activity = normalized EA.

Table 15 shows validation results of the type-2 scoring model. The same dataset as for validation of the type-1 scoring model was used: GSE76275 containing Basal-like breast cancer samples with an active NFkB pathway.

**Table 15: Type 2-model. Ranking Basals normalized by NFkB log2odd.**

| **Array ID GSM...** | **CD** | | | **APOP** | | | **OS** | | | **NFkB** |
|---|---|---|---|---|---|---|---|---|---|---|
| | | **score** | **rank** | | **score** | **rank** | | **score** | **rank** | **log2odd** |
| 1974604 | 2.43 | 0.22 | 3 | 1.28 | 0.32 | 2 | 3.05 | 0.30 | 1 | 31.93 |
| 1974614 | 2.44 | 0.22 | 3 | 1.25 | 0.31 | 2 | 3.18 | 0.32 | 1 | 29.87 |
| 1974626 | 2.49 | 0.23 | 3 | 1.39 | 0.35 | 1 | 2.78 | 0.28 | 2 | 29.76 |
| 1974593 | 2.50 | 0.23 | 3 | 1.51 | 0.38 | 2 | 3.47 | 0.35 | 1 | 28.62 |
| 1974567 | 2.77 | 0.25 | 3 | 1.33 | 0.33 | 2 | 3.35 | 0.33 | 1 | 27.85 |
| 1974738 | 2.86 | 0.26 | 3 | 1.53 | 0.38 | 1 | 3.30 | 0.33 | 2 | 26.90 |
| 1974592 | 2.97 | 0.27 | 3 | 1.62 | 0.41 | 1 | 3.40 | 0.34 | 2 | 25.95 |
| 1974724 | 2.86 | 0.26 | 3 | 1.40 | 0.35 | 2 | 3.26 | 0.33 | 1 | 24.74 |
| 1974721 | 3.08 | 0.28 | 3 | 1.54 | 0.39 | 2 | 3.98 | 0.40 | 1 | 24.65 |
| 1974747 | 3.26 | 0.30 | 3 | 1.51 | 0.38 | 2 | 3.69 | 0.37 | 1 | 24.12 |
| 1974710 | 3.22 | 0.29 | 3 | 1.71 | 0.43 | 1 | 3.46 | 0.35 | 2 | 23.43 |
| 1974628 | 3.13 | 0.28 | 3 | 1.87 | 0.47 | 1 | 3.78 | 0.38 | 2 | 22.85 |
| 1974605 | 3.76 | 0.34 | 3 | 1.38 | 0.34 | 2 | 4.21 | 0.42 | 1 | 22.34 |
| 1974696 | 3.37 | 0.31 | 3 | 1.77 | 0.44 | 2 | 4.22 | 0.42 | 1 | 21.88 |
| 1974659 | 3.50 | 0.32 | 3 | 1.81 | 0.45 | 1 | 4.02 | 0.40 | 2 | 20.87 |
| 1974595 | 3.65 | 0.33 | 3 | 2.06 | 0.52 | 1 | 4.08 | 0.41 | 2 | 20.84 |
| 1974744 | 3.63 | 0.33 | 3 | 1.96 | 0.49 | 1 | 4.17 | 0.42 | 2 | 20.33 |
| 1974732 | 3.92 | 0.36 | 3 | 1.95 | 0.49 | 1 | 4.28 | 0.43 | 2 | 20.31 |
| 1974591 | 3.66 | 0.33 | 3 | 1.94 | 0.49 | 1 | 4.32 | 0.43 | 2 | 20.23 |
| 1974692 | 3.79 | 0.34 | 3 | 2.16 | 0.54 | 1 | 4.24 | 0.42 | 2 | 19.44 |
| 1974718 | 4.01 | 0.36 | 3 | 2.01 | 0.50 | 1 | 4.45 | 0.45 | 2 | 19.11 |
| 1974749 | 4.16 | 0.38 | 3 | 1.96 | 0.49 | 2 | 4.66 | 0.47 | 1 | 17.76 |
| 1974670 | 4.44 | 0.40 | 3 | 2.18 | 0.55 | 1 | 4.49 | 0.45 | 2 | 17.07 |
| 1974691 | 4.22 | 0.38 | 3 | 2.48 | 0.62 | 1 | 4.77 | 0.48 | 2 | 16.94 |
| 1974664 | 4.32 | 0.39 | 3 | 2.06 | 0.52 | 2 | 4.99 | 0.50 | 1 | 16.86 |
| 1974712 | 4.53 | 0.41 | 3 | 2.41 | 0.60 | 1 | 4.74 | 0.47 | 2 | 16.71 |
| 1974679 | 4.34 | 0.39 | 3 | 2.20 | 0.55 | 1 | 4.61 | 0.46 | 2 | 16.29 |
| **average** | 3.46 | 0.31 | 3 | 1.79 | 0.45 | 1.41 | 3.96 | 0.40 | 1.59 | |

According to the type 2-model, NFkB activity-normalized score with subsequent NFkB function ranking. Normalized EA is obtained by dividing EA by NFkB pathway activity (as described above). Similar to (A) only the sample to analyze is required to calculate the EA score and rank the NFkB functions for the individual sample.

### Type 3-model. Subtraction of baseline gene expression level from the activated sample gene expression level prior to calculating EA.

In yet another model (type 3-model), instead of using absolute gene expression to calculate the EA score, the expression level of each FATG is first corrected for (i.e. substracted by) the baseline level of the respective FATGs as follows: for each FATG, the mRNA expression is determined for the sample to be analyzed and a reference sample. The reference sample is a cell sample, preferably of the same cell/tissue type as the sample to be analyzed, with low or no NFkB activity. Then, the differential expression is determined for each FATG by subtracting the baseline expression (expression level of reference sample) from the sample expression. The EA can then be calculated with the same equation as shown above. Contrary to the aforementioned models this method requires at least two samples (sample with active NFkB and reference sample with baseline activity).

Table 16 shows validation results of the type-3 scoring model. The same dataset as for validation of the other scoring models was used: GSE76275 containing Basal-like breast cancer samples with an active NFkB pathway.

**Table 16: Type 3-model. Ranking Basals based on differential expression (mean low NFkB luminal AR samples from same dataset subtracted).**

| **Array ID GSM...** | **CD** | | | **APOP** | | | **OS** | | | **NFkB** |
|---|---|---|---|---|---|---|---|---|---|---|
| | | **score** | **rank** | | **score** | **rank** | | **score** | **rank** | **log2odd** |
| 1974604 | 2.80 | 0.25 | 3 | 4.25 | 1.06 | 2 | 36.45 | 3.64 | 1 | 31.93 |
| 1974614 | -2.10 | -0.19 | 3 | 0.57 | 0.14 | 2 | 32.39 | 3.24 | 1 | 29.87 |
| 1974626 | -0.85 | -0.08 | 3 | 4.73 | 1.18 | 2 | 20.04 | 2.00 | 1 | 29.76 |
| 1974593 | -3.36 | -0.31 | 3 | 6.52 | 1.63 | 2 | 38.87 | 3.89 | 1 | 28.62 |
| 1974567 | 2.28 | 0.21 | 2 | 0.21 | 0.05 | 3 | 33.57 | 3.36 | 1 | 27.85 |
| 1974738 | 2.00 | 0.18 | 3 | 4.52 | 1.13 | 2 | 27.44 | 2.74 | 1 | 26.90 |
| 1974592 | 2.17 | 0.20 | 3 | 5.40 | 1.35 | 2 | 27.40 | 2.74 | 1 | 25.95 |
| 1974724 | -4.00 | -0.36 | 2 | -2.17 | -0.54 | 3 | 16.53 | 1.65 | 1 | 24.74 |
| 1974721 | 1.00 | 0.09 | 3 | 1.27 | 0.32 | 2 | 36.27 | 3.63 | 1 | 24.65 |
| 1974747 | 3.68 | 0.33 | 2 | -0.28 | -0.07 | 3 | 26.42 | 2.64 | 1 | 24.12 |
| 1974710 | 0.62 | 0.06 | 3 | 3.25 | 0.81 | 2 | 17.53 | 1.75 | 1 | 23.43 |
| 1974628 | -3.41 | -0.31 | 3 | 5.98 | 1.50 | 2 | 24.15 | 2.42 | 1 | 22.85 |
| 1974605 | 9.19 | 0.84 | 2 | -5.94 | -1.49 | 3 | 31.85 | 3.19 | 1 | 22.34 |
| 1974696 | -1.07 | -0.10 | 3 | 2.07 | 0.52 | 2 | 30.60 | 3.06 | 1 | 21.88 |
| 1974659 | -1.73 | -0.16 | 3 | 1.02 | 0.26 | 2 | 21.72 | 2.17 | 1 | 20.87 |
| 1974595 | 1.20 | 0.11 | 3 | 6.21 | 1.55 | 2 | 22.85 | 2.28 | 1 | 20.84 |
| 1974744 | -0.98 | -0.09 | 3 | 3.02 | 0.75 | 2 | 22.25 | 2.23 | 1 | 20.33 |
| 1974732 | 4.73 | 0.43 | 3 | 2.82 | 0.71 | 2 | 24.46 | 2.45 | 1 | 20.31 |
| 1974591 | -0.78 | -0.07 | 3 | 2.52 | 0.63 | 2 | 24.13 | 2.41 | 1 | 20.23 |
| 1974692 | -1.27 | -0.12 | 3 | 5.20 | 1.30 | 2 | 18.81 | 1.88 | 1 | 19.44 |
| 1974718 | 1.74 | 0.16 | 3 | 1.65 | 0.41 | 2 | 21.55 | 2.16 | 1 | 19.11 |
| 1974749 | -0.95 | -0.09 | 2 | -1.90 | -0.47 | 3 | 20.01 | 2.00 | 1 | 17.76 |
| 1974670 | 0.98 | 0.09 | 3 | 0.47 | 0.12 | 2 | 13.01 | 1.30 | 1 | 17.07 |
| 1974691 | -3.34 | -0.30 | 3 | 5.31 | 1.33 | 2 | 17.32 | 1.73 | 1 | 16.94 |
| 1974664 | -1.94 | -0.18 | 2 | -1.99 | -0.50 | 3 | 20.77 | 2.08 | 1 | 16.86 |
| 1974712 | 0.92 | 0.08 | 3 | 3.44 | 0.86 | 2 | 16.43 | 1.64 | 1 | 16.71 |
| 1974679 | -4.19 | -0.38 | 3 | -0.88 | -0.22 | 2 | 10.91 | 1.09 | 1 | 16.29 |
| **average** | 0.12 | 0.01 | 2.78 | 2.12 | 0.53 | 2.22 | 24.21 | 2.42 | 1.00 | |

According to the type 3-model, EA score is corrected for differences in background expression levels of the individual FATG genes, as described before, with subsequent ranking. The method requires, in addition to the sample to be analyzed, also a reference sample with low/absent NFkB activity, preferably of the same cell/tissue type. In this example the average of each target gene of a set of basal like breast cancer samples from the same dataset with a low NFkB activity are taken and subtracted from the expression level of corresponding gene of the basal sample. This differential value is entered in the EA equation, followed by ranking of the NFkB functions.

### Type 4-model. Subtraction of 'average expression of activated samples + baseline expression /2' from the activated sample activity, to calculate the EA.

In yet another model (type 4-model), to correct the FATG EA score for both the baseline level of the respective FATG and the varying magnitude of the expression increase upon activation, for each FATG the sum of 0.5 times the baseline expression (expression level of reference sample) and the averaged expression of activated samples is subtracted from the expression level of the sample to be analyzed. The EA can then be calculated based on this difference using the same equation as shown above.

Table 17 shows validation results of the type-4 scoring model. The same dataset as for validation of the other scoring models was used: GSE76275 containing Basal-like breast cancer samples with an active NFkB pathway.

**Table 17: Type 4-model. Ranking Basals based on differential score corrected for differences in expression level (average of mean low NFkB luminal AR and basals (below) subtracted).**

| **Array ID GSM...** | **CD** | | | **APOP** | | | **OS** | | | **NFkB** |
|---|---|---|---|---|---|---|---|---|---|---|
| | | **score** | **rank** | | **score** | **rank** | | **score** | **rank** | **log2odd** |
| 1974604 | 3.80 | 0.35 | 3 | 6.37 | 1.59 | 2 | 36.65 | 3.66 | 1 | 31.93 |
| 1974614 | -1.09 | -0.10 | 3 | 2.69 | 0.67 | 2 | 32.59 | 3.26 | 1 | 29.87 |
| 1974626 | 0.15 | 0.01 | 3 | 6.86 | 1.71 | 2 | 20.24 | 2.02 | 1 | 29.76 |
| 1974593 | -2.36 | -0.21 | 3 | 8.65 | 2.16 | 2 | 39.07 | 3.91 | 1 | 28.62 |
| 1974567 | 3.28 | 0.30 | 3 | 2.33 | 0.58 | 2 | 33.77 | 3.38 | 1 | 27.85 |
| 1974738 | 3.00 | 0.27 | 3 | 6.64 | 1.66 | 2 | 27.65 | 2.76 | 1 | 26.90 |
| 1974592 | 3.17 | 0.29 | 3 | 7.53 | 1.88 | 2 | 27.61 | 2.76 | 1 | 25.95 |
| 1974724 | -3.00 | -0.27 | 3 | -0.05 | -0.01 | 2 | 16.74 | 1.67 | 1 | 24.74 |
| 1974721 | 2.00 | 0.18 | 3 | 3.40 | 0.85 | 2 | 36.48 | 3.65 | 1 | 24.65 |
| 1974747 | 4.68 | 0.43 | 3 | 1.84 | 0.46 | 2 | 26.63 | 2.66 | 1 | 24.12 |
| 1974710 | 1.62 | 0.15 | 3 | 5.37 | 1.34 | 2 | 17.73 | 1.77 | 1 | 23.43 |
| 1974628 | -2.41 | -0.22 | 3 | 8.10 | 2.03 | 2 | 24.35 | 2.44 | 1 | 22.85 |
| 1974605 | 10.19 | 0.93 | 2 | -3.82 | -0.96 | 3 | 32.06 | 3.21 | 1 | 22.34 |
| 1974696 | -0.06 | -0.01 | 3 | 4.19 | 1.05 | 2 | 30.81 | 3.08 | 1 | 21.88 |
| 1974659 | -0.72 | -0.07 | 3 | 3.15 | 0.79 | 2 | 21.92 | 2.19 | 1 | 20.87 |
| 1974595 | 2.20 | 0.20 | 3 | 8.33 | 2.08 | 2 | 23.05 | 2.31 | 1 | 20.84 |
| 1974744 | 0.02 | 0.00 | 3 | 5.14 | 1.29 | 2 | 22.46 | 2.25 | 1 | 20.33 |
| 1974732 | 5.73 | 0.52 | 3 | 4.95 | 1.24 | 2 | 24.67 | 2.47 | 1 | 20.31 |
| 1974591 | 0.22 | 0.02 | 3 | 4.64 | 1.16 | 2 | 24.34 | 2.43 | 1 | 20.23 |
| 1974692 | -0.27 | -0.02 | 3 | 7.32 | 1.83 | 2 | 19.01 | 1.90 | 1 | 19.44 |
| 1974718 | 2.74 | 0.25 | 3 | 3.78 | 0.94 | 2 | 21.75 | 2.18 | 1 | 19.11 |
| 1974749 | 0.05 | 0.00 | 3 | 0.22 | 0.06 | 2 | 20.22 | 2.02 | 1 | 17.76 |
| 1974670 | 1.98 | 0.18 | 3 | 2.59 | 0.65 | 2 | 13.22 | 1.32 | 1 | 17.07 |
| 1974691 | -2.34 | -0.21 | 3 | 7.43 | 1.86 | 2 | 17.53 | 1.75 | 1 | 16.94 |
| 1974664 | -0.94 | -0.09 | 3 | 0.13 | 0.03 | 2 | 20.97 | 2.10 | 1 | 16.86 |
| 1974712 | 1.92 | 0.17 | 3 | 5.56 | 1.39 | 2 | 16.64 | 1.66 | 1 | 16.71 |
| 1974679 | -3.18 | -0.29 | 3 | 1.24 | 0.31 | 2 | 11.11 | 1.11 | 1 | 16.29 |
| **average** | 1.13 | 0.10 | 2.96 | 4.24 | 1.06 | 2.04 | 24.42 | 2.44 | 1.00 | |

According to the type 4 model, EA score is based on subtraction of the average levels of gene expression of a group of samples with an active NFkB pathway + the average of a group with low/absent NFkB activity (reference sample / baseline expression), divided by 2, and the expression of the gene in the sample to analyze, with subsequent calculation of EA score and ranking. In this example, the sum of the average of basal like breast samples and the average of an AR-positive subset of basal-like breast cancer samples (from the same dataset with a low NFkB activity) for each target gene divided by two is subtracted from the expression value of individual basal-like subtype breast cancer sample to correct both baseline variation of the different genes and mitigate effect of differences between gene expressions between individual samples.

### Application in the context of a clinical decision support (CDS) system

As will have become apparent from the present disclosure, the invention can be advantageously used to support clinical decisions, and may thus be integrated into a clinical decision support (CDS) system. With reference to Fig. 1, a clinical decision support (CDS) system 10 is implemented as a suitably configured computer 12. The computer 12 may be configured to operate as the CDS system 10 by executing suitable software, firmware, or other instructions stored on a non-transitory storage medium (not shown), such as a hard drive or other magnetic storage medium, an optical disk or another optical storage medium, a random access memory (RAM), a read-only memory (ROM), a flash memory, or another electronic storage medium, a network server, or so forth. While the illustrative CDS system 10 is embodied by the illustrative computer 12, more generally the CDS system may be embodied by a digital processing device or an apparatus comprising a digital processor configured to perform clinical decision support methods as set forth herein. For example, the digital processing device may be a handheld device (e.g., a personal data assistant or smartphone running a CDS application), a notebook computer, a desktop computer, a tablet computer or device, a remote network server, or so forth. The computer 12 or other digital processing device typically includes or is operatively connected with a display device 14 via which information including clinical decision support recommendations are displayed to medical personnel. The computer 12 or other digital processing device typically also includes or is operatively connected with one or more user input devices, such as an illustrative keyboard 16, or a mouse, a trackball, a trackpad, a touch-sensitive screen (possibly integrated with the display device 14), or another pointer-based user input device, via which medical personnel can input information such as operational commands for controlling the CDS system 10, data for use by the CDS system 10, or so forth.

The CDS system 10 receives as input information pertaining to a subject (e.g., a hospital patient, or an outpatient being treated by an oncologist, physician, or other medical personnel, or a person undergoing cancer screening or some other medical diagnosis who is known or suspected to have a certain type of immune-related disease and/or cancer, or a predisposition for developing an immune-related disease and/or cancer. The CDS system 10 applies various data analysis algorithms to this input information in order to generate clinical decision support recommendations that are presented to medical personnel via the display device 14 (or via a voice synthesizer or other device providing human-perceptible output). In some embodiments, these algorithms may include applying a clinical guideline to the patient. A clinical guideline is a stored set of standard or "canonical" treatment recommendations, typically constructed based on recommendations of a panel of medical experts and optionally formatted in the form of a clinical "flowchart" to facilitate navigating through the clinical guideline. In various embodiments the data processing algorithms of the CDS 10 may additionally or alternatively include various diagnostic or clinical test algorithms that are performed on input information to extract clinical decision recommendations, such as machine learning methods disclosed herein.

In the illustrative CDS systems disclosed herein (e.g., CDS system 10), the CDS data analysis algorithms include one or more diagnostic or clinical test algorithms that are performed on input genomic and/or proteomic information acquired by one or more medical laboratories 18. These laboratories may be variously located "on-site", that is, at the hospital or other location where the subject is undergoing medical examination and/or treatment, or "off-site", e.g., a specialized and centralized laboratory that receives (via mail or another delivery service) a sample of the subject that has been extracted from the subject.

The sample is processed by the laboratory to generate expression levels and optionally further genomic or proteomic information. For example, the sample may be processed using a microarray (also variously referred to in the art as a gene chip, DNA chip, biochip, or so forth) or by quantitative polymerase chain reaction (qPCR) processing to measure probative genomic or proteomic information such as expression levels of genes of interest (i.e. one or more FATGs), for example in the form of a level of messenger ribonucleic acid (mRNA) that is transcribed from the gene, or a level of a protein that is translated from the mRNA transcribed from the gene.

In some embodiments, the sample may be processed by a gene sequencing laboratory to generate sequences for deoxyribonucleic acid (DNA), or to generate an RNA sequence, copy number variation, methylation, or so forth. Other contemplated measurement approaches include immunohistochemistry (IHC), cytology, fluorescence in situ hybridization (FISH), proximity ligation assay or so forth, performed on a pathology slide. Other information that can be generated by microarray processing, mass spectrometry, gene sequencing, or other laboratory techniques includes methylation information. Various combinations of such genomic and/or proteomic measurements may also be performed.

In some embodiments, the medical laboratories 18 perform a number of standardized data acquisitions on the sample of the subject, so as to generate a large quantity of genomic and/or proteomic data. For example, the standardized data acquisition techniques may generate an (optionally aligned) DNA sequence for one or more chromosomes or chromosome portions, or for the entire genome. Applying a standard microarray can generate thousands or tens of thousands of data items such as expression levels for a large number of genes, various methylation data, and so forth. Similarly, PCR-based measurements can be used to measure the expression level of a selection of genes. This plethora of genomic and/or proteomic data, or selected portions thereof, are input to the CDS system 10 to be processed so as to develop clinically useful information for formulating clinical decision support recommendations.

The disclosed CDS systems and related methods relate to processing of genomic and/or proteomic data to assess expression level of certain function-associated target genes (FATGs) of an active NFkB cellular signaling pathway and to determine a cellular function of an active NFkB cellular signaling pathway in a cell sample. However, it is to be understood that the disclosed CDS systems (e.g., CDS system 10) may optionally further include diverse additional capabilities, such as generating clinical decision support recommendations in accordance with stored clinical guidelines based on various patient data such as vital sign monitoring data, patient history data, patient demographic data (e.g., gender, age, or so forth), patient medical imaging data, or so forth. Alternatively, in some embodiments the capabilities of the CDS system 10 may be limited to only performing genomic and/or proteomic data analyses to assess the expression level of certain function-associated target genes (FATGs) of an active NFkB cellular signaling pathway and to determine a cellular function of an active NFkB cellular signaling pathway in a cell sample, as disclosed herein.

With continuing reference to exemplary Fig. 1, the CDS system 10 determines a score 22 for each cellular function (e.g. expression average for apoptosis, cell division and oxidative stress protection; EA_A, EA_D, EA_F) based on expression levels 20 of one or more respective function-associated target genes of an active NFkB cellular signaling pathway activity measured in the sample of the subject.

The determined scores 22 are then used for differentiation 24 between the evaluated cellular functions in order to determine the most predominant cellular function 24 (or provide a ranking as disclosed herein) and/or provide additional information 28 relating to clinical recommendation based on the determined cellular function.

**SEQUENCE LISTING:**

| Seq. ID No.: | Gene: |
|---|---|
| Seq. ID No. 1 | BCL2 |
| Seq. ID No. 2 | BCL2L1 |
| Seq. ID No. 3 | BIRC3 |
| Seq. ID No. 4 | CCL2 |
| Seq. ID No. 5 | CCL5 |
| Seq. ID No. 6 | CCND2 |
| Seq. ID No. 7 | CSF2 |
| Seq. ID No. 8 | CYP27B1 |
| Seq. ID No. 9 | GZMB |
| Seq. ID No. 10 | IGHE / IGHG1 (could not be separated) |
| Seq. ID No. 11 | MMP1 |
| Seq. ID No. 12 | NOX1 |
| Seq. ID No. 13 | SERPINE2 |
| Seq. ID No. 14 | SKP2 |
| Seq. ID No. 15 | SOD2 |

## Claims

1. A method for determining a cellular function of an active NFkB cellular signaling pathway in a cell sample, the method comprising:
determining in the cell sample containing cells with an active NFkB cellular signaling pathway the cellular function based on expression level of at least one NFkB target gene associated with the cellular function.

2. The method according to claim 1,
wherein the at least one NFkB target gene is selected from the group consisting of BCL2L1, BCL2, CCND2, SKP2, NOX1, SERPINE2, CSF2, CYP27B1, IGHG1, IGHE, BIRC3, CCL2, SOD2, GZMB, CCL5 and MMP1, and
wherein the at least one NFkB target gene is preferably selected from the group consisting of BCL2L1, BCL2, CCND2, SKP2, CYP27B1, IGHG1, IGHE, BIRC3, CCL2, SOD2 and GZMB.

3. The method according to claim 1 or 2,
wherein the cellular function is selected from the group consisting of cell division, cell differentiation, cell adhesion, chemotaxis and migration, apoptosis, immune functions like antigen presentation, oxidative stress and oxidative stress protection, and
wherein the cellular function is preferably selected from the group consisting of cell division, apoptosis and oxidative stress protection.

4. The method according to one of the preceding claims,
wherein the at least one NFkB target gene is selectable based on differential expression studies comparing an expression level of the at least one cellular function-associated NFkB target gene of an active NFkB cellular signaling pathway, whose respective cellular function is active, with an expression level of the at least one cellular function-associated NFkB target gene of an active NFkB cellular signaling pathway, whose respective cellular function is inactive.

5. The method according to one of the preceding claims,
wherein the cellular function is determined based on evaluating a mathematical model relating the expression level of the at least one NFkB target gene to the cellular function.

6. The method according to claim 5,
wherein the expression level of the at least one NFkB target gene is an expression level normalized by and/or corrected for one or more of the following:
activity of the NFkB cellular signaling pathway in the cell sample;
baseline expression level of the respective NFkB target gene(s); and/or
average expression levels of the respective NFkB target gene(s) in a group of samples comprising cells with an active NFkB pathway.

7. The method according to one of the preceding claims,
wherein the determining of the cellular function involves discriminating between the cellular function and at least one further cellular function based on expression levels of target genes associated with the respective cellular functions.

8. The method according to one of the preceding claims,
wherein the cell sample is derived from a tissue, cells, blood, a body fluid, a cell line, a cell culture and/or a tissue culture.

9. The method according to one of the preceding claims,
wherein the cells contained in the cell sample are selected from the group consisting of immune cells and cancer cells and/or wherein the cells are obtained from a subject having, or suspected of having, or having a predisposition of acquiring, an immune-related condition such as kidney transplantation, rheumatoid arthritis, psoriasis, diabetes and preferably cancer.

10. The method according to one of the preceding claims,
wherein the cell sample containing cells with an active NFkB cellular signaling pathway is selected based on activity of the NFkB cellular signaling pathway in the cells contained in the cell sample,
wherein the activity of the NFkB cellular signaling pathway in the cells is inferred or inferable by a method comprising:
receiving expression levels of one or more and preferably six or more target genes of the NFkB cellular signaling pathway,
determining an level of an NFkB transcription factor (TF) element, the NFkB TF element controlling transcription of the one or more and preferably six or more target genes of the NFkB cellular signaling pathway, the determining being based at least in part on evaluating a mathematical model relating expression levels of the one or more and preferably six or more target genes of the NFkB cellular signaling pathway to the level of the NFkB TF element, wherein the one or more and preferably six or more target genes are selected from the group consisting of: BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TNIP 1, TRAF 1 and VCAM 1;
inferring the activity of the NFkB cellular signaling pathway based on the determined level of the NFkB TF element in the sample,
wherein the inferring is performed by a digital processing device using the mathematical model.

11. The method according to one of the preceding claims,
further comprising one or more of the following:
monitoring the at least one cellular function of the active NFkB cellular signaling pathway;
monitoring an effect of a therapy, preferably an immunotherapy, on the at least one cellular function of the active NFkB pathway;
identifying a mechanism of a drug targeting the NFkB cellular signaling pathway;
predicting or monitoring response to a drug targeting the NFkB cellular signaling pathway; and/or
predicting whether immune cells exert a tumor suppressive or a tumor promoting effect;
predicting therapy response, in particular response to immunotherapy;
predicting response to a combination immunotherapy as compared to monotherapy,
predicting whether a therapy, in particular an immunotherapy, is effective in view of side effects and/or costs; and/or
identifying patients that are at risk of severe side-effects.

12. An apparatus for determining at least one cellular function of an active NFkB cellular signaling pathway comprising at least one digital processor configured to perform the method of any of claims 1 to 11.

13. A non transitory storage medium for determining at least one cellular function of an active NFkB cellular signaling pathway storing instructions that are executable by a digital processing device to perform the method of any of claims 1 to 11.

14. A computer program for determining at least one cellular function of an active NFkB cellular signaling pathway comprising program code means for causing a digital processing device to perform a method of any of claims 1 to 10, when the computer program is run on the digital processing device.

15. A kit for use in a method of diagnosis of a mammal, the diagnosis being based on determining at least one cellular function of an active NFkB cellular signaling pathway in the mammal, the kit comprising:
components for determining the expression levels of at least one NFkB target gene associated with a cellular function, and optionally
the apparatus of claim 12, the non-transitory storage medium of claim 13, or the computer program of claim 14.
